# EUROPÄISCHE PATENTANMELDUNG

(11) **EP 2 149 352 A1**
(43) Veröffentlichungstag der Anmeldung: **03.02.2010**
(21) Anmeldenummer: 08013613.8
(22) Anmeldetag: 29.07.2008
(51) Int. Cl.: A61F 2/30, A61F 2/44

(54) **Zwischenwirbelimplantat**

(71) Anmelder: DERU GmbH, 22844 Norderstedt (DE)
(72) Erfinder: Link, Helmut D., 22397 Hamburg (DE)
(74) Vertreter: Glawe, Delfs, Moll

(57) **Zusammenfassung**

Die Erfindung betrifft ein Zwischenwirbelimplantat mit einer Anschlussfläche (12), die im implantierten Zustand des Zwischenwirbelimplantats an einer Oberfläche eines Wirbelkörpers anliegt. Das Implantat weist eine Mehrzahl von gegenüber der Anschlussfläche (12) vorspringenden Zähnen (13), deren von der Anschlussfläche (12) abgewandtes Ende in einer Kante (19) ausläuft. Erfindungsgemäß ist die Kante (19) abgerundet, wobei der Radius (r) der Kante (19) nicht kleiner ist als 0,1 mm. Wenn die Zähne (13) des erfindungsgemäßen Implantats in den Knochen eines Wirbelkörpers eindringen, so wird das Knochengewebe nicht durchtrennt, sondern lediglich verdichtet. Der Halt des Implantats in dem Wirbelkörper ist verbessert.

## Beschreibung

Die Erfindung betrifft ein Zwischenwirbelimplantat zum Einsetzen in einen Zwischenwirbelraum. Das Zwischenwirbelimplantat umfasst eine Anschlussfläche, die dazu ausgelegt ist, an einer den Zwischenwirbelraum begrenzenden Oberfläche eines Wirbelkörpers anzuliegen. Auf der Anschlussfläche ist eine Mehrzahl von Zähnen angeordnet, die gegenüber der Anschlussfläche vorspringen. Das von der Anschlussfläche abgewandte Ende der Zähne läuft in einer Kante aus.

Früher wurden Zwischenwirbelimplantate meist mechanisch mit dem Wirbelkörper verbunden wurden, beispielsweise indem ein Flansch des Implantats an dem Wirbelkörper festgeschraubt wurde. Zunehmend werden Implantate beispielsweise durch Aufbringen einer porösen Beschichtung auf den entsprechenden Oberflächenanteilen so ausgebildet, dass das Knochengewebe eine innige Verbindung mit der Oberfläche des Implantats aufbauen kann. Die innige Verbindung vermittelt dem Implantat Halt, auf eine zusätzliche mechanische Verbindung kann verzichtet werden.

Bislang war es das Ziel, dass die innige Verbindung zwischen dem Knochengewebe und der Oberfläche des Implantats so stabil und so haltbar wie möglich ist. Dem liegt die Annahme zu Grunde, dass das Implantat für den Rest des Lebens im Körper bleiben soll. Es gibt dann keine anderen Anforderungen an die Verbindung zwischen dem Implantat und dem Knochen, außer dass sie hinreichend fest sein muss, um allen denkbaren Belastungssituationen gewachsen zu sein.

Zunehmend werden Implantate bei jüngeren Patienten eingesetzt, deren Lebenserwartung größer ist als die zu erwartende Lebensdauer des Implantats. Es muss von vornherein damit gerechnet werden, dass es zu einem späteren Zeitpunkt erforderlich wird, das Implantat wieder von dem Knochen zu lösen, um es durch ein neues zu ersetzen.

Ist das Knochengewebe eine innige Verbindung mit der Oberfläche des Implantats eingegangen, so wird man beim Herauslösen des Implantats versuchen, mit einem scharfen Werkzeug zwischen den Knochen und das Implantat zu fahren, um die Verbindung aufzubrechen. Dies ist ohne größere Schwierigkeiten möglich, wenn die Oberfläche des Implantats eine ebene oder nur leicht gewölbte Form hat. Wesentlich schwieriger wird das Aufbrechen der Verbindung, wenn aus einer solchen Fläche Zähne herausragen, die in das Knochenmaterial eingedrungen sind und die in der Tiefe des Knochens mit dem Knochengewebe verbunden sind. In die Tiefe des Knochens kann man der Chirurg nämlich mit seinen Werkzeugen nicht vordringen.

Ist die Oberfläche der Zähne so gestaltet, dass das Knochengewebe eine innige Verbindung aufbauen kann, so leisten die Zähne ohne weiteres ihren Beitrag zu einer festen Verbindung zwischen dem Knochen und dem Implantat. Möchte man jedoch auf eine innige Verbindung zwischen dem Knochengewebe und der Oberfläche der Zähne verzichten, um ein späteres Herauslösen des Implantats zu erleichtern, so ist es nicht ohne weiteres sichergestellt, dass die Zähne eine ausreichenden Beitrag zur Fixierung des Implantats leisten.

Ausgehend vom eingangs genannten Stand der Technik liegt der Erfindung die Aufgabe zu Grunde, ein Zwischenwirbelimplantat vorzustellen, bei dem die Zähne so gestaltet sind, dass sie dem Implantat auch ohne eine innige Verbindung zum Knochengewebe einen guten Halt im Knochen vermitteln. Die Aufgabe wird gelöst durch die Merkmale des unabhängigen Anspruchs. Erfindungsgemäß ist die von der Anschlussfläche abgewandte Kante des Zahns abgerundet, wobei der Radius der Kante nicht kleiner ist als 0,1 mm. Vorteilhafte Ausführungsformen finden sich in den Unteransprüchen.

Zunächst werden einige Begriffe erläutert. Eine Kante entsteht dort, wo zwei Flächen aufeinanderstoßen. Die Kante hat eine Längsausdehnung, die parallel zu jeder der beiden Flächen ausgerichtet ist. Eine Kante an einem von der Anschlussfläche abgewandten Ende des Zahns hat über ihre gesamte Länge einen Abstand zu der Anschlussfläche. Die Kante ist quer zu ihrer Längsausdehnung abgerundet.

Die erfindungsgemäß abgerundeten Zähne dringen mit ihrem gesamten Volumen in das Knochengewebe ein, so dass die Anschlussfläche, gegenüber der die Zähne vorspringen, flächig auf der Oberfläche des Knochengewebes aufliegt. Indem die zuerst in das Knochengewebe eindringende Kante der Zähne nicht scharf, sondern abgerundet ist, wird das Knochengewebe beim Eindringen der Zähne nicht durchtrennt, sondern lediglich verdichtet. Das rund um die Zähne herum verdichtete Knochengewebe nimmt Druckkräfte von den Zähnen besser auf, als ein Knochengewebe, das durch das Eindringen eines scharfkantigen Zahns geschwächt ist.

Die Zähne dienen in erster Linie dazu, über ihre Flanken Druckkräfte auf das Knochengewebe zu übertragen, die im wesentlichen parallel zur Anschlussfläche wirken. Zugkräfte, die in einer zur Anschlussfläche senkrechten Richtung wirken, sollen vorwiegend von der Anschlussfläche selbst auf das Knochengewebe übergeleitet werden. Dazu kann die Anschlussfläche mit einer porösen Beschichtung belegt sein, so dass das Knochengewebe in die Poren einwachsen kann und dadurch eine innige Verbindung mit der Anschlussfläche eingeht. Die nach ASTM F1854 bestimmte Porenweite kann zwischen 30 µm und 70 µm liegen, der Porenanteil zwischen 20% und 40%. Da zwischen der Oberfläche der Zähne und dem Knochengewebe keine innige Verbindung entstehen soll, bleiben die Zähne vorzugsweise von der Beschichtung ausgespart. Die Flächen der Zähne, die an die abgerundete Kante angrenzen, sind also nicht porös. Ferner sollte die Oberfläche der Zähne auch keine zu stark ausgeprägte Rauigkeit haben. Vorzugsweise ist die gemäß DIN EN ISO 4288 und 3274 bestimmte Rauigkeit Rₐ in kleiner als 12 µm, weiter vorzugsweise kleiner als 8 µm. Ein Zwischenwirbelimplantat, bei dem die Zähne von der Beschichtung ausgespart sind, und bei dem die unbeschichteten Spitzen der Zähne aus der Beschichtung herausragen, könnte eine eigenständige Erfindung sein, auch ohne dass die Kanten der Zähne abgerundet sind.

Das erfindungsgemäße Zwischenwirbelimplantat wird vorzugsweise im zervikalen Bereich der Wirbelsäule eingesetzt. Um nicht übermäßig in dem Wirbelkörper einzugreifen, sollte die Höhe der Zähne über der Anschlussfläche kleiner als 1,5 mm, vorzugsweise kleiner als 1,2 mm sein. Diese Angaben beziehen sich auf den Körper des Implantats. Ist auf den Körper des Implantats eine Beschichtung aufgebracht, so ist die Höhe der Zähne gegenüber der Beschichtung entsprechend der Dicke der Beschichtung vermindert. Die Höhe der Zähne sollte 0,8 mm nicht unterschreiten. Der Radius der abgerundeten Kante liegt dann in einer Größenordnung zwischen 5% und 15% der Höhe der Zähne. Die Breite der Zähne ist vorzugsweise kleiner als 3 mm, weiter vorzugsweise kleiner als 2 mm.

Beim Einsetzen eines erfindungsgemäßen Zwischenwirbelimplantats verschafft sich der Chirurg von der ventralen Seite her Zugang zur Wirbelsäule. Mittels eines Werkzeugs, das am ventralen Ende des Zwischenwirbelimplantats angreift, wird das Implantat in dorsaler Richtung in den Zwischenwirbelraum eingesetzt. Die Richtung zwischen dem ventralen und dem dorsalen Ende der Prothese wird als AP-Richtung bezeichnet. Rechtwinklig dazu ist die laterale Richtung.

Um das Einsetzen des Implantats in den Zwischenwirbelraum zu erleichtern, kann die in dorsaler Richtung weisende Fläche der Zähne eine geringere Steigerung gegenüber der Anschlussfläche haben als die in ventraler Richtung weisende Fläche der Zähne. Die Steigung dieser dorsalen Flanke gegenüber der Anschlussfläche kann beispielsweise zwischen 40° und 60° liegen. Aus dem Stand der Technik sind Zähne dieser Art bekannt, bei denen die der dorsalen Flanke gegenüberliegende ventrale Flanke der Zähne, die einem Herausziehen des Implantats in ventraler Richtung entgegenwirkt, senkrecht auf der Anschlussfläche steht. Zwar ist eine möglichst senkrecht stehende Flanke vorteilhaft für eine gute Kraftübertragung in ventraler Richtung, es hat sich jedoch bei der Entwicklung der Erfindung gezeigt, dass Zähne mit einer senkrecht zur Anschlussfläche ausgerichteten Flanke dazu neigen, Knochengewebe zu durchtrennen, wenn sie in den Knochen eindringen. Die Gefahr einer Beschädigung des Knochengewebes kann im Rahmen der Erfindung dadurch vermindert werden, dass die ventrale Flanke nicht senkrecht auf der Anschlussfläche steht, sondern unter einem Winkel zwischen 78° und 88°, vorzugsweise zwischen 80° und 85°. Die gleiche Erwägung gilt für die lateralen Flanken der Zähne. Wiederum können die Zähne schonender in das Knochengewebe eindringen, wenn die lateralen Flanken nicht senkrecht, sondern unter einem Winkel von etwas weniger als 90° auf der Anschlussfläche stehen. Der bevorzugte Winkelbereich ist derselbe wie bei der ventralen Flanke.

Die Anschlussfläche des Zwischenwirbelimplantats kann in AP-Richtung betrachtet eben sein. Die Anschlussfläche kann auch entsprechend der Form eines benachbarten Wirbelkörpers in AP-Richtung gewölbt sein.

Wenn die Zähne lediglich das Knochengewebe verdichten, ohne eine innige Verbindung mit dem Knochengewebe einzugehen, so kommt der Anschlussfläche eine umso größere Bedeutung für die stabile Verbindung zwischen dem Implantat und dem Knochen zu. Erstrebenswert ist deswegen eine möglichst große Anschlussfläche. Betrachtet man die horizontale Kontur des erfindungsgemäßen Zwischenwirbelimplantats, so wird diese abgesehen von den Zähnen vorzugsweise vollständig von der Anschlussfläche eingenommen. Zumindest sollten 80%, vorzugsweise 90% der neben den Zähnen verbleibenden Fläche von der Anschlussfläche eingenommen werden. Die Zähne sind vorzugsweise so angeordnet, dass die zusammenhängenden Bereiche der Anschlussfläche zwischen den Zähnen möglichst groß sind. Beispielsweise kann eine Mehrzahl von Zähnen in einer sich im wesentlichen in AP-Richtung erstreckenden Reihe angeordnet sein. Vorzugsweise sind die Zähne so klein, dass sie in ihrer Summe nicht mehr als 15%, vorzugsweise nicht mehr als 10%, weiter vorzugsweise nicht mehr als 8% der Fläche einnehmen, die von der Kontur des Zwischenwirbelimplantats eingeschlossen ist. Mit allen diesen Maßnahmen wird erreicht, dass die Fläche, über die das Knochengewebe eine innige Verbindung mit der Anschlussfläche eingehen kann, möglichst groß ist.

Der Körper des erfindungsgemäßen Zwischenwirbelimplantats kann aus einem gängigen Material, wie beispielsweise einer Metalllegierung oder einem Keramikwerkstoff, bestehen.

Die Erfindung wird nachfolgend unter Bezugnahme auf die beigefügten Zeichnungen anhand einer vorteilhaften Ausführungsform beispielhaft beschrieben. Es zeigen:
- Fig. 1: eine Ansicht von oben auf ein erfindungsgemäßes Zwischenwirbelimplantat;
- Fig. 2: einen Schnitt entlang der Linie A-A in Fig. 1;
- Fig. 3: einen Schnitt entlang der Linie B-B in Fig. 1;
- Fig. 4: einen vergrößerten Ausschnitt aus Fig. 3; und
- Fig. 5: die Ansicht aus Fig. 4 mit angedeutetem Knochengewebe.

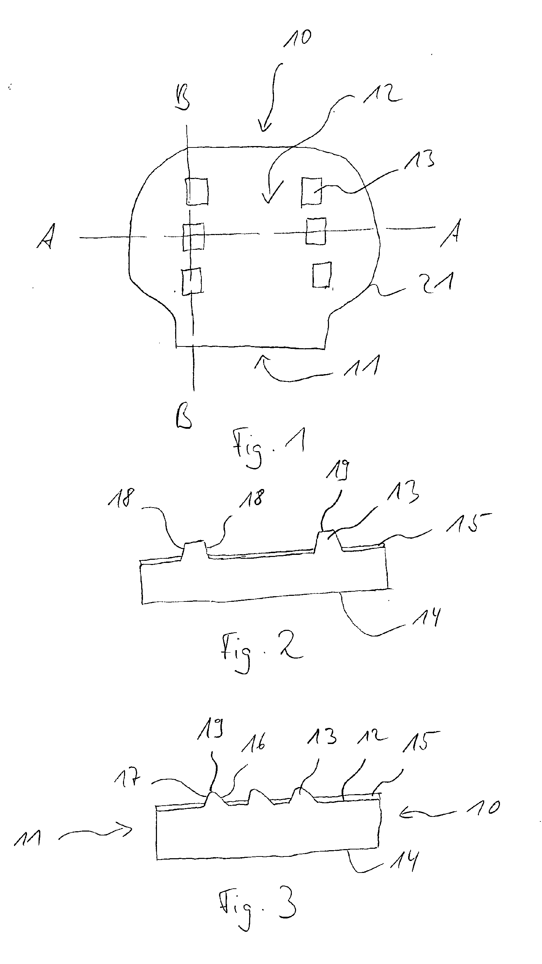

Ein Fig. 1 gezeigtes Zwischenwirbelimplantat ist dazu ausgelegt, mit seinem dorsalen Ende 10 voran in einen Zwischenwirbelraum eingesetzt zu werden. Beim Einsetzen wird das Zwischenwirbelimplantat mit einem Instrument gehandhabt, das am ventralen Ende 11 des Zwischenwirbelimplantats angreift. Ist das Implantat in den Zwischenwirbelraum eingesetzt, liegt eine Anschlussfläche 12 an einer den Zwischenwirbelraum begrenzenden Oberfläche eines Wirbelkörpers an. Das erfindungsgemäße Implantat kann mit weiteren Prothesenkomponenten zusammenwirken, um beispielsweise insgesamt eine Zwischenwirbelprothese zu bilden, die die Funktion einer Bandscheibe nachbildet. Dazu kann die der Anschlussfläche 12 gegenüberliegende Unterseite 14 des Implantats als Gleitfläche ausgebildet sein, die mit einer Gleitfläche der anderen Prothesenkomponente ein Gelenk bildet.

Auf der Anschlussfläche 12 sind sechs Zähne 13 ausgebildet, die gegenüber der Anschlussfläche 12 vorspringen. Die Zähne 13 sind in zwei im wesentlichen in AP-Richtung ausgerichteten Reihen zu je drei Zähnen 13 angeordnet. Ist das Implantat in den Zwischenwirbelraum eingesetzt, dringen die Zähne 13 in das Knochengewebe des benachbarten Wirbelkörpers ein, so dass die Anschlussfläche 12 auf der Oberfläche des Wirbelkörpers aufliegt. Die Anschlussfläche 12 ist mit einer Schicht 15 belegt, mit der das Knochengewebe eine innige Verbindung eingehen kann. Die Zähne 13 sind von der Schicht 15 ausgespart. Abgesehen von den Zähnen 13 erstreckt sich die Anschlussfläche 12 über die gesamte von der Kontur 21 des Implantats eingeschlossene Fläche.

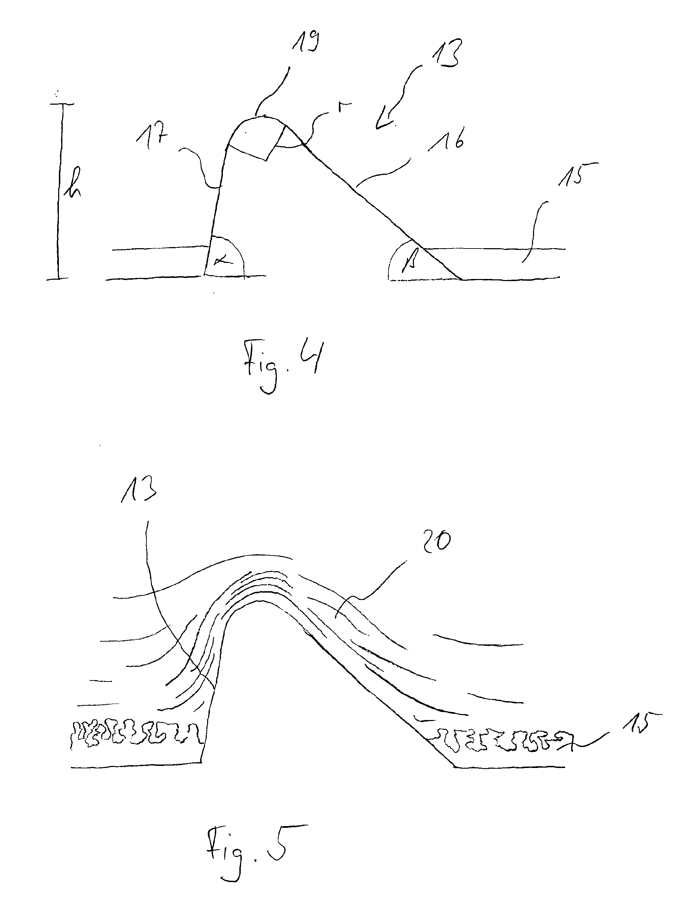

Jeder Zahn 13 hat eine dorsale Flanke 16, eine ventrale Flanke 17 sowie zwei laterale Flanken 18. Die dorsale Flanke 16 geht über eine abgerundete Kante 19 in die ventrale Flanke 17 über. Die Kante 19 erstreckt sich über die Breite des Zahns 13 von einer lateralen Flanke 18 bis zur anderen lateralen Flanke 18. Die ventrale Flanke 17 des Zahns 13 hat eine in Fig. 4 durch den Winkel α angedeutete Steigung von 82° gegenüber der horizontalen Ebene des Implantats. Die durch den Winkel β angedeutete Steigung der dorsalen Flanke 16 ist ungefähr 40°. Die lateralen Flanken 18 haben genau wie die ventrale Flanke 17 eine Steigung von 82° gegenüber der horizontalen Ebene. Die Zähne 13 haben eine Höhe h von 1,1 mm gegenüber der Anschlussfläche 12. Aus der 0,2 mm dicken Beschichtungen 15 ragen die Zähne um 0,9 mm heraus. Die Abrundung der Kante 19 hat einem Radius r von 0,1 mm.

Die Beschichtung 15 ist, wie in Fig. 5 angedeutet, so porös, dass das ebenfalls in Fig. 5 angedeutete Knochengewebe 20 des Wirbelkörpers eine innige Verbindung mit der Beschichtung 15 eingehen kann. Durch den eindringenden Zahn 13 wird das Knochengewebe 20 nicht durchtrennt, sondern lediglich komprimiert.

## Patentansprüche

1. Zwischenwirbelimplantat mit einer Anschlussfläche (12), die im implantierten Zustand des Zwischenwirbelimplantats an einer Oberfläche eines Wirbelkörpers anliegt, und mit einer Mehrzahl von gegenüber der Anschlussfläche (12) vorspringenden Zähnen (13), deren von der Anschlussfläche (12) abgewandtes Ende in einer Kante (19) ausläuft, **dadurch gekennzeichnet, dass** die Kante (19) abgerundet ist, wobei der Radius (r) der Kante (19) nicht kleiner ist als 0,1 mm.

2. Zwischenwirbelimplantat nach Anspruch 1, **dadurch gekennzeichnet, dass** die Anschlussfläche (12) mit einer porösen Beschichtung (15) belegt ist und dass die Zähne (13) von der Beschichtung (15) ausgespart sind.

3. Zwischenwirbelimplantat nach Anspruch 1 oder 2, **dadurch gekennzeichnet, dass** die Rauigkeit Rₐ der über die Kante (19) verbundenen Flanken (16, 17) der Zähne (13) kleiner als 12 µm, vorzugsweise kleiner als 8 µm ist.

4. Zwischenwirbelimplantat nach einem der Ansprüche 1 bis 3, **dadurch gekennzeichnet, dass** die Höhe (h) der Zähne (13) über der Anschlussfläche (12) kleiner als 1,5 mm, vorzugsweise kleiner als 1,2 mm ist.

5. Zwischenwirbelimplantat nach einem der Ansprüche 1 bis 4, **dadurch gekennzeichnet, dass** die Steigung (β) der dorsalen Flanke (16) der Zähne (13) gegenüber der Anschlussfläche (12) geringer ist als die Steigung (α) der ventrale Flanke (17) der Zähne (13).

6. Zwischenwirbelimplantat nach einem der Ansprüche 1 bis 5, **dadurch gekennzeichnet, dass** die ventrale Flanke (17) der Zähne (13) eine Steigung (α) zwischen 78° und 88°, vorzugsweise zwischen 80° und 85° gegenüber der Anschlussfläche (12) hat.

7. Zwischenwirbelimplantat nach einem der Ansprüche 1 bis 6, **dadurch gekennzeichnet, dass** die lateralen Flanken (18) der Zähne (13) eine Steigung von weniger als 90° gegenüber der Anschlussfläche (12) haben.

8. Zwischenwirbelimplantat nach einem der Ansprüche 1 bis 7, **dadurch gekennzeichnet, dass** die Anschlussfläche (12) in AP-Richtung gewölbt ist.

9. Zwischenwirbelimplantat nach einem der Ansprüche 1 bis 8, **dadurch gekennzeichnet, dass** die Anschlussfläche (12) sich abgesehen von den Zähnen (13) über die gesamte von der Kontur (21) des Zwischenwirbelimplantats eingeschlossene Fläche erstreckt.

10. Zwischenwirbelimplantat nach einem der Ansprüche 1 bis 9, **dadurch gekennzeichnet, dass** eine Mehrzahl von Zähnen (13) in einer sich im wesentlichen in AP-Richtung erstreckenden Reihe angeordnet ist.

11. Zwischenwirbelimplantat nach einem der Ansprüche 1 bis 10, dass die Zähne (13) nicht mehr als 15 %, vorzugsweise nicht mehr als 10 %, weiter vorzugsweise nicht mehr als 8 % der der von der Kontur (21) des Zwischenwirbelimplantats eingeschlossenen Fläche einnehmen.
